# EUROPÄISCHE PATENTSCHRIFT

(11) **EP 4 178 662 B1**
(45) Veröffentlichungstag und Bekanntmachung des Hinweises auf die Patenterteilung: **03.06.2026**
(21) Anmeldenummer: 21723648.8
(22) Anmeldetag: 21.04.2021
(51) Int. Cl.: A61M 60/00

(54) **BLUTPUMPE**
BLOOD PUMP
POMPE À SANG

(30) Priorität: 07.07.2020 DE 102020117818
(43) Veröffentlichungstag der Anmeldung: 17.05.2023
(73) Patentinhaber: ResuSciTec GmbH, 79098 Freiburg (DE)
(72) Erfinder: BENK, Christoph, 79098 Freiburg (DE); HEIMGARTNER, Lion, 79098 Freiburg (DE)
(74) Vertreter: Rösler, Uwe
(86) Internationale Anmeldenummer: PCT/EP2021/060363
(87) Internationale Veröffentlichungsnummer: WO 2022/008114

(56) Entgegenhaltungen:
- DE-A1- 102006 036 948
- DE-A1- 19 626 224
- US-A1- 2017 361 001

## Beschreibung

### Technisches Gebiet

Die Erfindung bezieht sich auf eine Blutpumpe mit einem Pumpengehäuse, das einen flaschenhalsförmigen Strömungseintrittsbereich umfasst, in dem eine an einer drehbar gelagerten Drehwelle drehfest angebrachte Schaufelanordnung angeordnet ist, die mit dem Pumpengehäuse einen Hauptdurchströmungskanal einschliesst und eine Anzahl n radial zur Drehwelle orientierte, jeweils ebenflächig ausgebildete Schaufelblätter besitzt, die äquidistant zueinander um die Drehwelle angeordnet sind. Stromab an den flaschenhalsförmigen Strömungseintrittsbereich schließt sich ein hohlzylinderförmiger Pumpengehäuseabschnitt an, längs dem ein im Wesentlichen orthogonal zum Strömungseintritt orientierter Strömungsauslass und in dem innerhalb eines Innengehäuses ein Drehmotor angeordnet sind, der über eine Magnetkupplung mit der Schaufelanordnung zu deren Drehantrieb in Wirkverbindung steht. Das Innengehäuse schließt mit dem Pumpengehäuse einen den Hauptdurchströmungskanal weiterführenden Durchflusskanal sowie mit der Schaufelanordnung einen mit dem Hauptdurchströmungskanal fluidisch kommunizierenden Sekundärkanal ein, der mit wenigstens einem die Schaufelanordnung durchsetzenden und in den flaschenhalsförmigen Strömungseintrittsbereich mündenden Spülkanal fluidisch verbunden ist. Das Innengehäuse sieht zudem zur Schaufelanordnung zugewandt ein unteres Lager zur drehbeweglichen Aufnahme eines unteren Endes der Drehwelle vor, deren oberes Ende in einer im flaschenhalsförmigen Strömungseintrittsbereich angeordneten Lagerhülse gelagert ist.

### Stand der Technik

Aus der Druckschrift DE 196 26 224 A1 ist eine Blutpumpe zu entnehmen, in deren Pumpengehäuse eine Schaufelanordnung angeordnet ist, die gemeinsam mit einer Drehwelle um eine das Pumpengehäuse zentrisch durchsetzende Drehachse drehbar gelagert ist. Die Drehwelle ist an ihren Drehwellenenden an einem oberen und unteren Lager drehbar gefasst. Das untere Lager sitzt an der Oberseite eines Innengehäuses auf, das stromab zur Schaufelanordnung innerhalb des Pumpengehäuses angeordnet ist, und umschließt einen elektromotorischen Drehantrieb, der über eine Magnetkupplung mit der drehfest an der Drehwelle angeordneten Schaufelanordnung in Wirkverbindung steht.

Die schraubenförmig gestalteten Schaufelräder der Schaufelanordnung schließen mit der Innenwand des Pumpengehäuses einen axialen Hauptdurchströmungskanal ein, durch den druckgetrieben eine Blutströmung durch die Blutpumpe hervorgerufen wird. Zudem weist die Schaufelanordnung einen Sekundär- oder Spülkanal auf, der die Saugseite der Schaufelanordnung mit deren Rückseite hydraulisch verbindet. Auf diese Weise kann Blut in einen rückseitigen Spalt zwischen der Schaufelanordnung und dem Innengehäuse gelangen und diesen spülen. Totwasserbereiche an der Rückseite der Schaufelanordnung können so wirksam vermieden werden.

Die Druckschrift US 2018/0050140 A1 offenbart eine Blutpumpe mit einem Impeller, der schneckenartig gestaltete Schaufeln vorsieht und von sechs, sogenannten Waschkanälen durchsetzt ist, die mindestens eine tangentiale Richtungskomponente besitzen und im Wesentlichen einen kreisrunden Kanalquerschnitt besitzen.

Aus der Druckschrift EP 1727 987 B1 ist eine Blutpumpe zu entnehmen, mit einer innerhalb des Pumpengehäuses über eine Magnetlagerung drehbar gelagerte Schaufelanordnung, deren Schaufeln ebenflächig, schneckenförmig oder anderweitig geschwungen ausgebildet sein können. Durch die Magnetlagerung der Schaufelanordnung ist eine kostengünstige und verschleißarme Pumpe zu realisieren, die insbesondere eine zum Pumpengehäuse kontaktfreie Lagerung der Schaufelanordnung vorsieht. Durch das Fehlen von mit dem Pumpengehäuse verbundenen Lagerstreben kann die Problematik der Blutgerinnselbildung im Zuströmungsbereich der Blutpumpe zumindest verringert werden. Die bekannte Schaufelanordnung stützt sich lediglich über ein Kugellager ab, das an einem den Drehmotor umfassendes Innengehäuse angebracht ist.

Eine vergleichbare Blutpumpe mit Magnetlagerung der Schaufelanordnung ist der Druckschrift EP 2 566 533 B1 zu entnehmen, die zur Abführung von betriebsbedingter Reibungswärme einen mit dem Innengehäuse innerhalb des Pumpengehäuses verbundenen metallischen, kegelförmig ausgebildeten Stift aufweist, auf dem über eine Kugelkontur die Schaufelanordnung drehbar gelagert ist.

Alle bekannten Blutpumpen, die auf dem Rotationsprinzip einer Schaufelanordnung beruhen, nutzen zum Fluss- und Druckaufbau innerhalb der Blutpumpe den zentrifugalen Effekt der rotierenden Schaufelanordnung, der neben einem Hauptförderstrom längs des von der Schaufelanordnung sowie dem Innengehäuse und dem Pumpengehäuse radial begrenzten Hauptdurchströmungskanal, eine Reihe von Sekundärströmungen hervorruft, die von der Hauptströmung abzweigen und von dort aus weitere Zwischenräume innerhalb der Pumpe durchströmen. Um zu vermeiden, dass der konstruktionsbedingte Zwischenspalt zwischen der Schaufelanordnung und dem, den Drehmotor umgebenden Innengehäuse keinen Strömungstotraum und eine damit verbundene Thrombenbildungsgefahr darstellt, weist die Schaufelanordnung einen sogenannten Spülkanal auf, durch den aufgrund der vorherrschenden Druckverhältnisse ein sekundärer Blutstrom den Zwischenspalt und den mit diesem fluidisch verbundenen Spülkanal durchströmt. Ohne besondere Vorkehrungen bildet sich durch den die Schaufelanordnung durchsetzenden Spülkanal eine zur Hauptdurchströmungsrichtung retrograde Sekundärströmung aus.

Die Druckschrift US 2017/0361001 A1 offenbart eine Zentrifugal-Blutpumpe, bei der sich der Strömungseinlass sowie auch der Strömungsauslass im gleichen Halbraum relativ zur rotierend gelagerten Schaufelanordnung befinden. Konstruktionsbedingt wird das Blut unmittelbar nach Eintritt in das bekannte Pumpengehäuse durch die Schaufelanordnung radial nach außen abgelenkt und tritt über ein oberhalb der Ebene der Schaufelanordnung angebrachten Strömungsauslass radial nach außen aus. In einer Ausführungsform sieht die Schaufelblattanordnung um die Drehachse sektoral ausgeformte Öffnungen vor, durch die Blut auch an die Unterseite der Schaufelblattanordnung gelangen kann. An der Unterseite der Schaufelblattanordnung vorgesehene, im Wesentlichen radial orientierte, nutförmig ausgebildete Strömungskanäle sorgen für einen Druckunterschied, durch den das an die Unterseite der Schaufelblattanordnung gelangte Blut radial nach außen und durch, in der Schaufelblattanordnung vorgesehene Durchtrittsöffnungen wieder in den oberen Bereich der Schaufelblattanordnung zurück fließt, um schließlich aus der Blutpumpe radial nach außen auszuströmen. Die um die Drehachse sektoral ausgeformten Öffnungen stellen keine Spülkanäle im vorstehend genannten Sinne, dar und werden auch nicht von einer retrograden Sekundärströmung durchsetzt.

Die Druckschrift DE 10 2006 036948 A1 offenbart eine Blutpumpe mit einer Schaufelanordnung mit Deckscheibe, die eine Magnetlagerung derart vorsieht, so dass auf ein oberes Lager für die Schaufelanordnung verzichtet werden kann. Die Schaufelanordnung sitzt drehbar auf einer zentralen Lagerachse auf, die zusammen mit der Schaufelanordnung einen ringförmigen Spülkanal begrenzt.

### Darstellung der Erfindung

Der Erfindung liegt die Aufgabe zugrunde, eine Blutpumpe mit einem Pumpengehäuse, das einen flaschenhalsförmigen Strömungseintrittsbereich umfasst, in dem eine an einer drehbar gelagerten Drehwelle drehfest angebrachte Schaufelanordnung angeordnet ist, die mit dem Pumpengehäuse einen Hauptdurchströmungskanal einschliesst und eine Anzahl n radial zur Drehwelle orientierte, jeweils ebenflächig ausgebildete Schaufelblätter besitzt, die äquidistant zueinander um die Drehwelle angeordnet sind, sowie einen sich stromab an den flaschenhalsförmigen Strömungseintrittsbereich anschließenden, hohlzylinderförmigen Pumpengehäuseabschnitt aufweist, längs dem ein im Wesentlichen orthogonal zum Strömungseintritt orientierter Strömungsauslass und in dem innerhalb eines Innengehäuses ein Drehmotor angeordnet sind, der über eine Magnetkupplung mit der Schaufelanordnung zu deren Drehantrieb in Wirkverbindung steht, und das Innengehäuse mit dem Pumpengehäuse einen den Hauptdurchströmungskanal weiterführenden Durchflusskanal sowie mit der Schaufelanordnung einen mit dem Hauptdurchströmungskanal fluidisch kommunizierenden Sekundärkanal einschließt, der mit wenigstens einem die Schaufelanordnung durchsetzenden und in den flaschenhalsförmigen Strömungseintrittsbereich mündenden Spülkanal fluidisch verbunden ist, und das Innengehäuse zudem der Schaufelanordnung zugewandt ein unteres Lager zur drehbeweglichen Aufnahme eines unteren Endes der Drehwelle vorsieht, deren oberes Ende in einer im flaschenhalsförmigen Strömungs-Eintrittsbereich angeordneten Lagerhülse lagert, derart weiterzubilden, dass der Wirkungsgrad der Blutpumpe verbessert werden soll, d.h. das mit Hilfe der Blutpumpe geförderte Blutvolumen soll mit einem geringeren Energieaufwand bewerkstelligt werden. Insbesondere gilt es, die Betriebsdrehzahl der drehbar angetriebenen Schaufelanordnung signifikant zu reduzieren, ohne dabei das geförderte Blutvolumen im Vergleich zu bekannten Blutpumpen zu verkleinern. Mit der anzustrebenden Maßnahme zum Zwecke einer ökonomischeren Betriebsweise der Blutpumpe und einer damit einhergehenden, wünschenswerten Reduzierung der Betriebsdrehzahl, soll darüber hinaus der mit Hilfe der Blutpumpe realisierte Bluttransport schonender bewerkstelligt werden.

Die Lösung der der Erfindung zugrunde liegenden Aufgabe ist im Anspruch 1 angegeben. Die lösungsgemäße Blutpumpe in vorteilhafter Weise ausbildende Merkmale sind Gegenstand der Unteransprüche sowie der weiteren Beschreibung insbesondere unter Bezugnahme auf das illustrierte Ausführungsbeispiel zu entnehmen.

Die lösungsgemäße Blutpumpe mit den im Oberbegriff des Anspruches 1 angegebenen Merkmalen zeichnet sich dadurch aus, dass drei separate Spülkanäle mit jeweils einer zur Drehwelle parallel orientierten Spülkanallängsachse gleich verteilt um die Drehwelle angeordnet sind und die drei Spülkanäle jeweils einen orthogonal zur Drehwelle orientierten Spülkanalquerschnitt besitzen, der jeweils nierenförmig ausgebildet ist und die Drehwelle sektoral umfasst.

Im Rahmen einer großen Vielzahl von Strömungsversuchen mit unterschiedlich konfektionierten Blutpumpen konnte das vorstehende Blutpumpendesign mit optimierten strömungsdynamischen Eigenschaften gefunden werden, die sich insbesondere darin widerspiegeln, dass durch die spezielle Wahl von Form und Anzahl der parallel zur Spülkanallängsachse orientierten Spülkanäle in Verbindung mit jeweils ebenflächig ausgebildeten Schaufelblättern, die sich radial zur Drehwelle erstrecken und jeweils zueinander einen um die Drehwelle äquidistanten Abstand aufweisen, bei einer vorgegebenen Drehzahl das größte Fördervolumen erzielt werden kann. Die lösungsgemäße Blutpumpe vermag anders ausgedrückt im Vergleich zu bekannten Blutpumpen gleicher Baugröße ein vorgegebenes Fördervolumen mit einer geringeren Drehzahl zu bewältigen, wodurch das durch die Blutpumpe hindurchströmende Blut einer geringeren mechanischen Belastung ausgesetzt ist.

Vorzugsweise sind die nierenförmigen Spülkanalquerschnitte jeweils von einem Umfangsrand umfasst, der eine radial äußere, von der Drehwelle abgewandte konvexe Umfangskontur und eine radial innere, zur Drehwelle zugewandte konkave Umfangskontur aufweist. Dabei liegen die konkaven Umfangskonturen der drei Spülkanalquerschnitte jeweils auf einer zentrisch zur Drehwelle angeordneten ersten virtuellen Kreislinie. Die konvexen Umfangskonturen der drei Spülkanalquerschnitte liegen hingegen auf einer zentrisch zur Drehwelle angeordneten zweiten virtuellen Kreislinie, deren Radius größer ist als der Radius der ersten Kreislinie.

Als besonders vorteilhaft hat es sich erwiesen sechs Schaufelblätter jeweils gleich verteilt um die Drehwelle anzuordnen.

In einer besonders bevorzugten Ausführungsform verfügen die jeweils sechs Schaufelblätter über eine radiale Erstreckung, die sich in axialer Projektion auf die Schaufelanordnung längs der Drehwelle von der vorstehend erwähnten zweiten virtuellen Kreislinie, die jeweils die konkaven Umfangskonturen der Spülkanalquerschnitte begrenzt, bis zu einer zentrisch zur Drehwelle angeordneten dritten virtuellen Kreislinie erstreckt, wobei der Radius der zweiten virtuellen Kreislinie kleiner ist als der Radius der dritten virtuellen Kreislinie. Vorzugsweise entspricht der Radius der dritten Kreislinie maximal dem Radius des kreisförmigen Umfangsrandes der Schaufelanordnung in axialer Projektion zur Drehwelle.

In einer alternativen Ausführungsform der Blutpumpe sind die sechs Schaufelblätter jeweils in zwei Gruppen, eine erste und eine zweite Gruppe hinsichtlich ihrer Form und Größe sowie auch Anordnung an der Schaufelanordnung unterteilt. So besitzen drei der sechs Schaufelblätter, die der ersten Gruppe angehören und im Weiteren als Primärschaufeln bezeichnet werden, jeweils eine radiale Erstreckung, die sich in axialer Projektion auf die Schaufelanordnung längs der Drehwelle von der ersten virtuellen Kreislinie, die die vorstehend erwähnten konkaven Umfangskonturen der nierenförmigen Spülquerschnitte begrenzt, bis zu einer zentrisch zur Drehwelle angeordneten dritten virtuellen Kreislinie erstreckt, die, wie in vorstehenden Fall maximal dem Radius des kreisförmigen Umfangsrandes der Schaufelanordnung entspricht.

Die drei der zweiten Gruppe zugehörigen Schaufelblätter, die sogenannten Sekundärschaufeln, besitzen hingegen jeweils eine radiale Erstreckung, die sich in axialer Projektion auf die Schaufelanordnung längs der Drehwelle von der zweiten virtuellen Kreislinie bis zu der zentrisch zur Drehwelle angeordneten dritten virtuellen Kreislinie erstreckt. Dabei sind die Primär- und Sekundärschaufeln jeweils mit abwechselnder Reihenfolge um die Drehwelle angeordnet. Weitere diesbezügliche konstruktive Merkmale und Ausgestaltungsdetails sind der weiteren Beschreibung unter Bezugnahme auf die Figuren zu entnehmen.

Vorzugsweise sind zudem das obere und untere Lager, in denen jeweils das obere bzw. untere Drehwellenende drehbar aber axial fest gefügt sind, aus einem keramischen oder abriebfesten Kunststoffmaterial, vorzugsweise UHM-Kunststoff gefertigt. Das am Innengehäuse angebrachte untere Lager sieht vorzugsweise ein fest mit dem Innengehäuse gefügtes, topfförmiges Inlay-Element vor, in das das untere Ende der Drehwelle drehbar und axial fest einmündet und aus einem anderen Material gefertigt ist, als jenes, aus dem das Innengehäuse selbst besteht.

### Kurze Beschreibung der Erfindung

Die Erfindung wird nachstehend ohne Beschränkung des allgemeinen Erfindungsgedankens anhand von bevorzugten Ausführungsbeispielen unter Bezugnahme auf die Zeichnungen exemplarisch beschrieben. Es zeigen:
- Fig. 1a, b: Seiten- und Draufsicht auf das Pumpengehäuse,
- Fig. 2: Längsschnittdarstellung durch das Pumpengehäuse im Bereich des Strömungseintrittes,
- Fig. 3: oberes Lagerelement,
- Fig. 4: Längsschnittdarstellung durch das Pumpengehäuse,
- Fig. 5: Draufsicht auf die Schaufelanordnung gemäß einer ersten Ausführungsform,
- Fig. 6: perspektivische Ansicht auf die Schaufelanordnung gemäß einer zweiten Ausführungsform
- Fig. 7: Draufsicht auf die Schaufelanordnung gemäß der zweiten Ausführungsform und
- Fig. 8a-c: Mehrseitenansichten einer Schaufelanordnung mit Deckplatte.

### Wege zur Ausführung der Erfindung, gewerbliche Verwendbarkeit

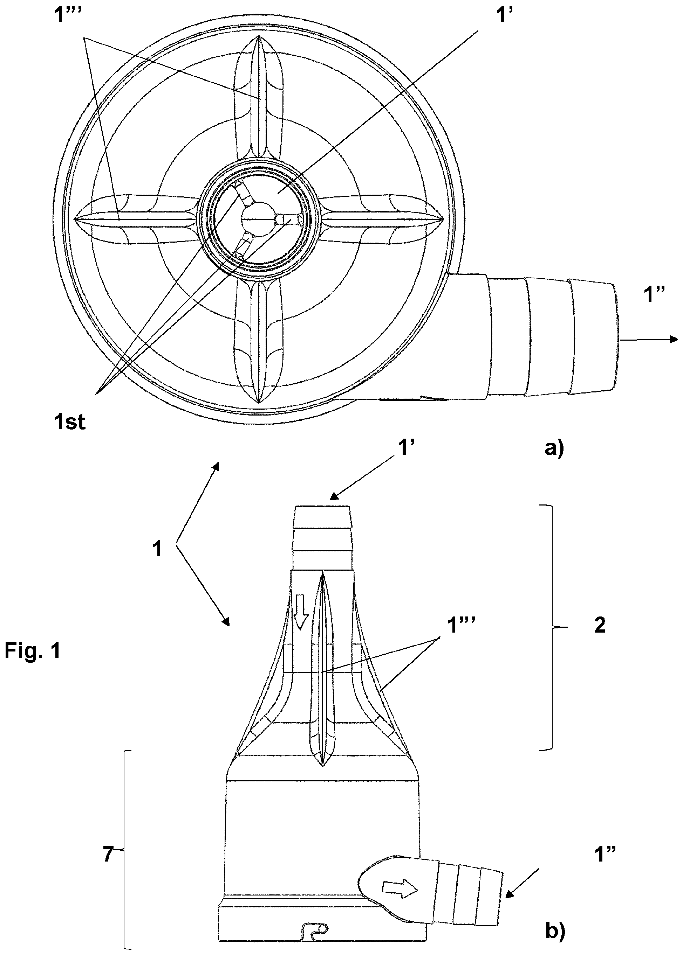

Die Figuren 1a und b zeigen eine Seiten- und Draufsicht auf das Pumpengehäuse 1 der Blutpumpe. Das im oberen Abschnitt im wesentlichen flaschenhalsförmig ausgebildete Pumpengehäuse 1 weist einen Strömungseintritt 1' auf, durch den Blut in Längserstreckung des Pumpengehäuses 1 in die Blutpumpe eintritt. Das Pumpengehäuse 1 ist unterteilbar in einen flaschenhalsförmigen Strömungseintrittsbereich 2 sowie einen sich daran bündig anschließenden hohlzylinderförmigen Pumpengehäuseabschnitt 7. Im Bereich des hohlzylinderförmigen Pumpengehäuseabschnittes 7 ist ein im Wesentlichen orthogonal zum Strömungseintritt orientierter Strömungsauslass 1" vorgesehen.

Um die Wandstärke des Pumpengehäuses 1 der Blutpumpe unter anderem aus Kosten- und Gewichtsgründen möglichst gering zu halten, sind vier Stabilitätsstreben 1‴ im Bereich des flaschenhalsförmigen Strömungseintrittsbereiches 2 an der Außenseite des Pumpengehäuses 1 angebracht.

Unmittelbar stromab des Strömungseintritts 1' sind mit der Innenwand des Pumpengehäuses 1 vier Stützstreben 1st einseitig verbunden, die orthogonal zu einer Art Einlaufstern zusammenlaufen aufeinander treffen und gemeinsam die räumliche Befestigung für das obere Drehwellenlager bilden, auf das im Weiteren eingegangen wird. Die Stützstreben 1st sind strömungsdynamisch optimiert ausgebildet, um einen möglichst geringen Strömungswiderstand für einen durch den Strömungseintritt 1' eintretenden Blutstrom zu bilden.

Die dargestellte Blutpumpe stellt eine Diagonalpumpe dar, d.h. der in Längserstreckung des Pumpengehäuses 1 durch den Strömungseintritt 1' eintretende Blutstrom wird orthogonal zur Einströmrichtung in einen tangential aus dem Pumpengehäuse 1 durch den Strömungsaustritt 1" austretenden Blutstrom umgeleitet.

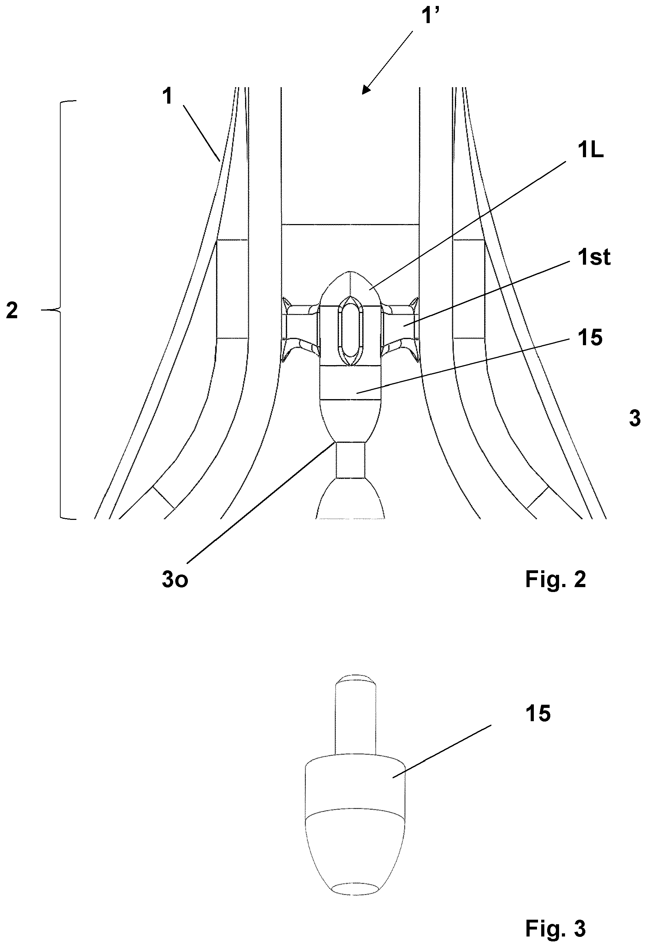

Figur 2 zeigt einen Teillängsschnitt durch den oberen Teil des Pumpengehäuses 1 im Bereich des flaschenhalsförmigen Strömungseintrittsbereiches 2 dar. Stromab des Strömungseintritts 1' befinden sich die mit dem Pumpengehäuse 1 einstückig verbundenen, strömungsdynamisch optimiert ausgebildeten Stützstreben 1st, die zu einer Art Einlaufstern zusammenlaufen und mittig an eine strömungsdynamisch günstige, kapselartig ausgebildete Lagerstruktur 1L münden. Vorzugsweise ist das Pumpengehäuse 1 samt der Stützstreben 1st sowie der Lagerstruktur 1L einstückig aus Kunststoff, vorzugsweise in einem Spritzgussverfahren hergestellt. Bündig, d.h. kantenlos, ist innerhalb der Lagerstruktur 1L eine Lagerhülse 15, vorzugsweise aus keramischem oder weitgehend verschleissfreiem Kunststoffmaterial eingebracht, in die das obere Ende der Drehwelle 3 axial fest und drehbar gefügt ist, siehe auch Figur 3.

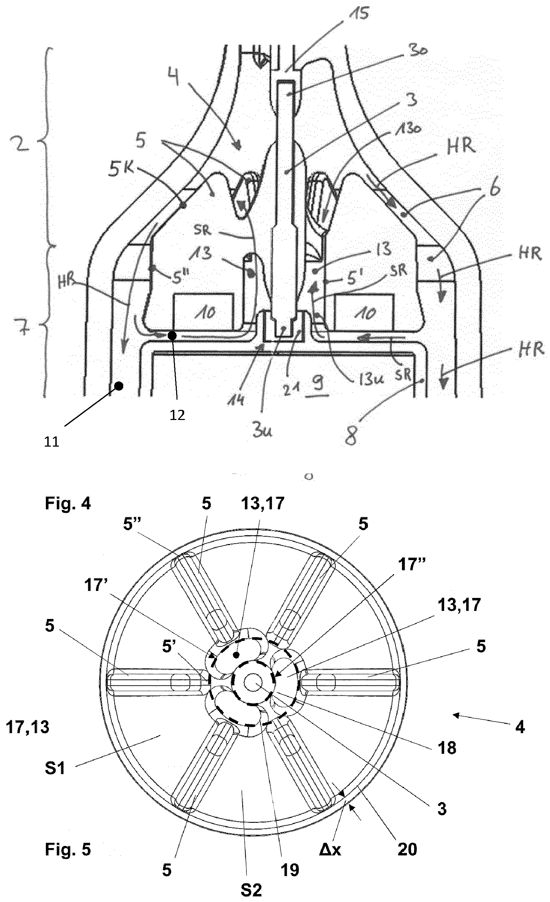

Figur 4 zeigt einen weiteren Teillängsschnitt durch das Pumpengehäuse 1, der den flaschenhalsförmigen Strömungseintrittsbereich 2 und einen Teil des sich daran anschliessenden hohlzylinderförmigen Pumpengehäuseabschnittes 7 darstellt.

Das Pumpengehäuse 1 umfasst eine Drehwelle 3, deren oberes Drehwellenende 3o in die Lagerhülse 15 axial fest und drehbar einmündet, eine drehfest mit der Drehwelle 3 verbundene Schaufelanordnung 4 sowie ein Innengehäuse 8, an dessen oberen Seite ein unteres Lager 14 mit einem Inlay-Element 21, in das das untere Drehwellenende 3u axial fest und drehbar gelagert ist.

Die drehfest mit der Drehwelle 3 verbundene Schaufelanordnung 4 weist sechs ebenflächig ausgebildete Schaufelblätter 5 auf, die in dem in Figur 4 dargestellten Ausführungsbeispiel in Form und Größe gleichartig ausgebildet sind. Die sechs Schaufelblätter 5 sind jeweils äquidistant zueinander um die Drehwelle 3 angeordnet. Ihre radial äußeren Schaufelblattkonturen schließen mit der Innenwand des Pumpengehäuses 1 einen Hauptströmungskanal 6 ein, durch den der Blutstrom in Hauptdurchströmungsrichtung HR bei Betrieb der Blutpumpe hindruchtritt.

Ergänzend an dieser Stelle sei auf die Figur 5 verwiesen, die die Schaufelanordnung 4 in axialer Draufsicht in Strömungsrichtung zeigt. Die Schaufelanordnung 4 schließt drei Spülkanäle 13 an, die gleich verteilt um die Drehwelle 3 angeordnet sind. Jeder der Spülkanäle 13 weist einen nierenförmig ausgebildeten Spülkanalquerschnitt 17 auf, der jeweils eine von der Drehwelle 3 abgewandte konvexe Umfangskontur 17' sowie eine radial innere, zur Drehwelle 3 zugewandte konkave Umfangskontur 17" aufweist.

Die jeweils konkaven Umfangskonturen 17" der drei Spülkanalquerschnitte 17 liegen auf einer zentrisch zur Drehwelle 3 angeordneten ersten virtuellen Kreislinie 18. Die konvexen Umfangskonturen 17' der jeweils drei Spülkanalquerschnitte 17 liegen hingegen auf einer zentrisch zur Drehwelle angeordneten zweiten virtuellen Kreislinie 19.

Sämtliche Schaufelblätter 5 weisen in dem dargestellten Ausführungsbeispiel jeweils radiale Erstreckungen auf, deren radial der Drehwelle 3 zugewandten Schaufelblattenden 5' allesamt in axialer Projektion auf die Schaufelanordnung 4 auf der virtuellen zweiten Kreislinie 19 liegen. Die jeweils radial äußeren Enden 5" der Schaufelblätter 5 liegen hingegen auf einer dritten virtuellen Kreislinie 20, die dem Umfangsrand der Schaufelanordnung 4 entspricht oder von diesem durch einen geringen Abstand Δx beabstandet ist, mit 0,1 mm <=δ x <= 2 mm.

Die drei Spülkanäle 13 erstrecken sich parallel zur Drehachse 3 und verfügen jeweils über eine strömungsdynamisch optimiert ausgeformte untere Spülkanalöffnung 13u sowie eine obere Spülkanalöffnung 13o.

Die jeweils obere Spülkanalöffnung 13o mündet in zwei Strömungsbereiche S1, S2, von denen jeder Strömungsbereich jeweils von zwei Schaufelblättern 5 begrenzt ist. Die jeweils orchideenkelchartig ausgeformten oberen Spülkanalöffnungen 13o bieten für den Fall einer zur längs des Hauptströmungskanals 6 durchströmenden Hauptströmung HR retrograd orientierten Spülkanalströmung SR einen geringst möglichen Strömungswiderstand, wodurch die retrograde Spülströmung SR weitgehend turbulenzfrei, jedoch zumindest turbulenzarm, im flaschenhalsförmigen Strömungseintrittsbereich 2 in die Hauptströmung HR einzumünden vermag.

Auch die untere Spülkanalöffnung 13u jedes Spülkanals 13 ist strömungsoptimiert für einen geringst möglichen Strömungswiderstand ausgebildet. Hierzu sieht das am Innengehäuse 8 angebrachte untere Lager 14 eine strömungsdynamisch in Richtung der unteren Spülkanalöffnung 13u orientierte Strömungskulisse vor, die für eine retrograde Spülkanalströmung SR einen geringst möglichen Eintrittswiderstand in die jeweiligen Spülkanäle 13 bildet.

Die Schaufelanordnung 4 verfügt darüber hinaus über eine Magnetkupplung 10, die in magnetischer Kopplung zu einem innerhalb des Innengehäuses 8 angebrachten Drehmotor 9 steht. Die für die Magnetkopplung 10 erforderlichen Permanentmagnete bzw. magnetischen Einheiten sind vollständig innerhalb der Schaufelanordnung 4 eingebracht, die vorzugsweise aus einem verschleissfreien Kunststoffmaterial besteht. Die Magneteinheiten können entweder im Rahmen eines Gießprozesses oder eines generativen Herstellungsverfahrens zur Erzeugung der Schaufelanordnung 4 vollständig verkapselt eingebracht werden.

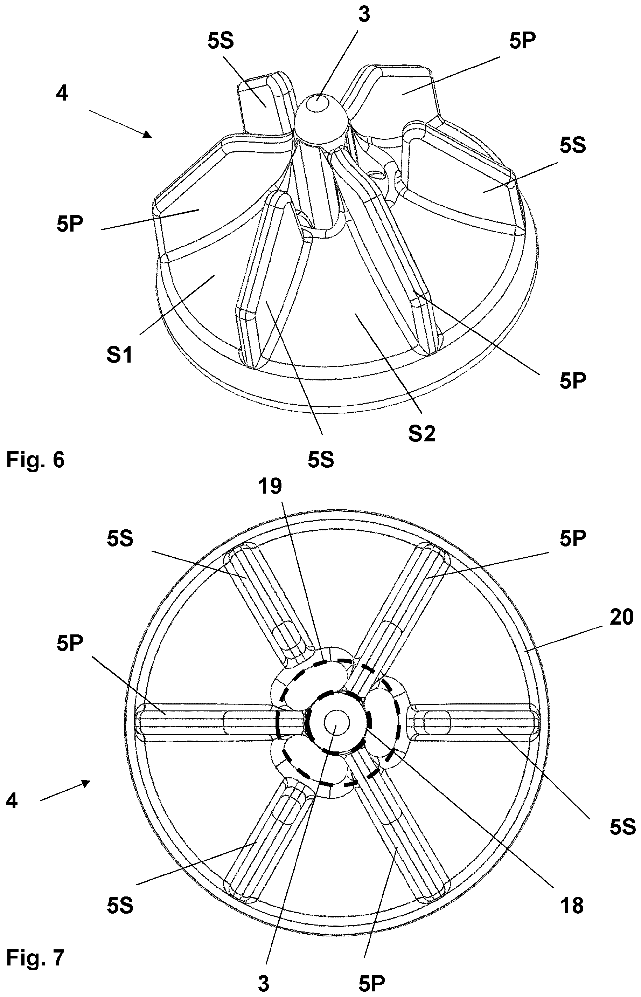

In den Figuren 6 und 7 ist eine alternative Ausführungsform für eine Schaufelanordnung 4 in einer perspektivischen Schrägansicht sowie einer axialen Draufsicht dargestellt. Im Unterschied zu der in den Figuren 4 und 5 gezeigten Schaufelanordnung weist die Schaufelanordnung 4 in den Figuren 6 und 7 jeweils drei in Form und Größe identisch ausgebildete Primärschaufeln 5P auf, die sich in der axialen Anordnung um die Drehwelle 3 mit radial kürzer ausgebildeten Sekundärschaufeln 5S abwechseln. Sämtliche Schaufeln 5P und 5S sind jeweils ebenflächig ausgebildet. Die Primärschaufeln 5P verfügen über eine radiale Erstreckung, die sich von der ersten virtuellen Kreislinie 18 bis zur randseitigen dritten Kreislinie 20 erstrecken. Die Sekundärschaufeln 5S erstrecken sich hingegen lediglich von der zweiten virtuellen Kreislinie 19 bis zur dritten Kreislinie 20.

Aus Figur 6 ist zu ersehen, dass die jeweils obere Spülkanalöffnung 13o sich orchideenkelchartig zwischen zwei Primärschaufeln 5P öffnet, wobei jeweils eine Sekundärschaufel 5S den oberen Öffnungsbereich in zwei Strömungsbereiche S1 und S2 aufteilt.

Die jeweils drei Primärschaufeln 5P münden an ihren radial zur Drehwelle 3 zugewandten Ende unmittelbar bzw. mittelbar an der Drehwelle 3. Die Sekundärschaufeln 5S verfügen mit Ausnahme ihrer verkürzten radialen Erstreckung ansonsten über eine gleichförmig und gleich dimensionierte ebenflächige Erstreckung wie die Primärschaufeln 5P.

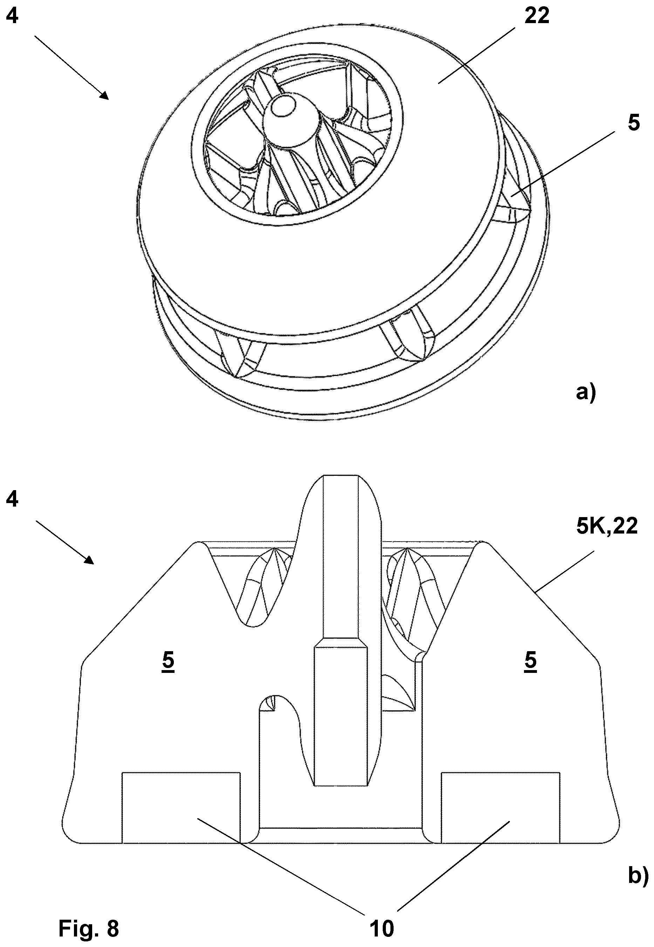

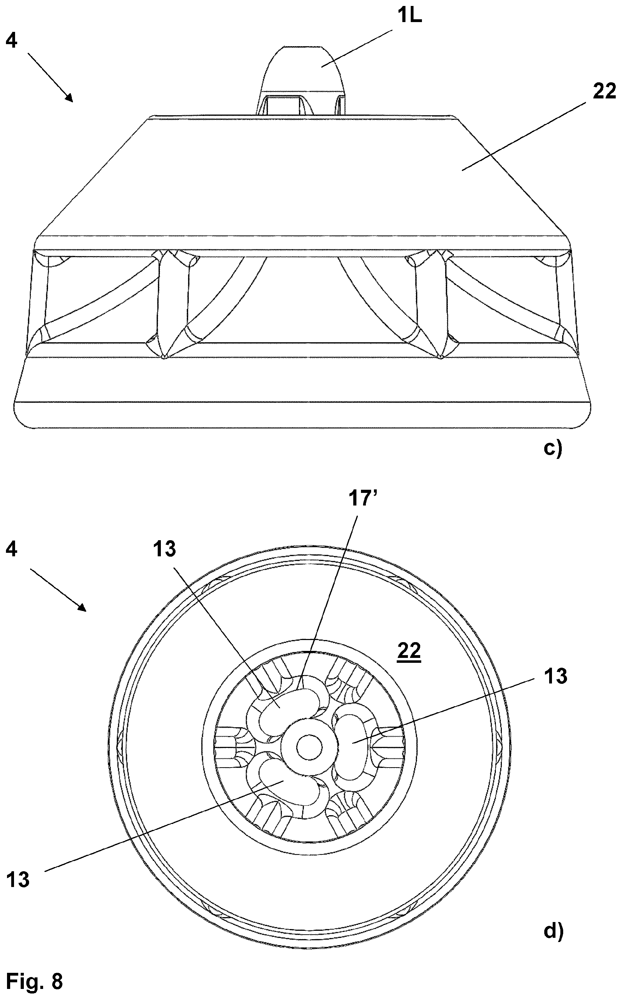

In den Figuren 8 a, b, c und d ist jeweils eine alternative Ausbildungsform zur Ausgestaltung der Schaufelanordnung 4 mit einer Deckscheibe 22 in verschiedenen Ansichten bzw. Darstellungsformen illustriert. Fig. 8a zeigt die Schaufelanordnung 4 in perspektivischer Darstellung von schräg oben, Fig. 8b als Längsschnitt, Fig. 8c von der Seite und Fig. 8d von oben. Die Schaufelanordnung 4 gleicht der Schaufelanordnung, die bereits in den Figuren 4 und 5 gezeigt ist, d.h. die einzelnen Schaufelblätter 5 sind alle identisch ausgebildet und grenzen mit ihren radial inneren Schaufelkanten 5' jeweils an die konvexen Umfangskonturen der 17' nierenförmigen Spülkanalquerschnitte 17. Zusätzlich sind die der Strömung zugewandten Seitenkanten 5k, siehe Figur 4, der Schaufelblätter 5 mit einer Deckscheibe 22 verbunden, die mit der Innenwand des Pumpengehäuses 1 den Hauptdurchströmungskanal 6 begrenzt. Die Deckscheibe 22 ist konisch ausgebildet und weist eine ansonsten gerad-Kegelstumpf-förmige Oberfläche auf, die mit der Innenwand des Pumpengehäuses 1 einen unabhängig von der Drehzahl der Schaufelanordnung 4 unveränderlich vorgegebenen Hauptdurchströmungskanal 6 begrenzt. Die Deckscheibe 22 trägt dazu bei die auf den Blutstrom bei Durchtritt durch die Blutpumpe wirkenden Scherkräfte und mechanischen Belastungen zu reduzieren, wodurch das Blut schonender die Blutpumpe passieren kann.

Selbstverständlich ist es auch möglich die Deckscheibe 22 an der Schaufelanordnung 4 gemäß der in den Figuren 6 und 7 dargestellten Ausführungsbeispielen entsprechend vorzusehen.

### Bezugszeichenliste

- 1: Pumpengehäuse
- 1': Strömungseintritt
- 1": Strömungsaustritt
- 1‴: Stabilitätsstreben
- 1st: Stützstreben
- 1L: Lagerstruktur
- 2: Flaschenhalsförmiger Strömungseintrittsbereich
- 3: Drehwelle
- 3o: Oberes Drehwellenende
- 3u: Unteres Drehwellenende
- 4: Schaufelanordnung
- 5: Schaufelblätter
- 5P: Primärschaufeln
- 5S: Sekundärschaufeln
- 5': Radial innere Schaufelkante
- 5": Radial äußere Schaufelkante
- 5K: Seitenkante
- 6: Hauptdurchströmungskanal
- 7: hohlzylinderförmiger Pumpengehäuseabschnitt
- 8: Innengehäuse
- 9: Drehmotor
- 10: Magnetkupplung
- 11: Weiterführender Durchflusskanal
- 12: Sekundärkanal
- 13: Spülkanal
- 13u: Untere Spülkanalöffnung
- 13o: Obere Spülkanalöffnung
- 14: Unteres Lager
- 15: Lagerhülse
- 16: Spülkanallängsachse
- 17: Spülkanalquerschnitt
- 17': Konvexe Umfangskontur
- 17": Konkave Umfangskontur
- 18: Erste Kreislinie
- 19: Zweite Kreislinie
- 20: Dritte Kreislinie
- 21: Inlay-Element
- 22: Deckscheibe
- SR: Sekundärströmung, Spülströmung
- HR: Hauptströmung
- S1, S2: Strömungsbereiche
- R1, R2, R3: Radien

## Patentansprüche

1. Blutpumpe mit einem Pumpengehäuse (1), das einen flaschenhalsförmigen Strömungseintrittsbereich (2) umfasst, in dem eine an einer drehbar gelagerten Drehwelle (3) drehfest angebrachte Schaufelanordnung (4) angeordnet ist, die mit dem Pumpengehäuse (1) einen Hauptdurchströmungskanal (6) einschliesst, durch den ein Blutstrom in Hauptdurchströmungsrichtung (HR) bei Betrieb der Blutpumpe hindurchtritt, und eine Anzahl n radial zur Drehwelle orientierte, jeweils ebenflächig ausgebildete Schaufelblätter (5) besitzt, die äquidistant zueinander um die Drehwelle (3) angeordnet sind, sowie einen sich stromab an den flaschenhalsförmigen Strömungseintrittsbereich (2) anschließenden, hohlzylinderförmigen Pumpengehäuseabschnitt (7) aufweist, längs dem ein im Wesentlichen orthogonal zum Strömungseintritt orientierter Strömungsauslass (1") und in dem innerhalb eines Innengehäuses (8) ein Drehmotor (9) angeordnet ist, der über eine Magnetkupplung (10) mit der Schaufelanordnung (4) zu deren Drehantrieb in Wirkverbindung steht, und das Innengehäuse (8) mit dem Pumpengehäuse (1) einen den Hauptdurchströmungskanal (6) weiterführenden Durchflusskanal (11) sowie mit der Schaufelanordnung (4) einen mit dem Hauptdurchströmungskanal (6) fluidisch kommunizierenden Sekundärkanal (12) einschließt, der mit wenigstens einem die Schaufelanordnung (4) durchsetzenden und in den flaschenhalsförmigen Strömungseintrittsbereich (2) mündenden Spülkanal (13) fluidisch verbunden ist, und das Innengehäuse (8) zudem der Schaufelanordnung (4) zugewandt ein unteres Lager (14) zur drehbeweglichen Aufnahme eines unteren Endes (3u) der Drehwelle (3) vorsieht, deren oberes Ende (3o) in einer im flaschenhalsförmigen Strömungseintrittsbereich angeordneten Lagerhülse (15), die mittelbar am Pumpengehäuse (1) befestigt ist, gelagert ist,
**dadurch gekennzeichnet, dass** drei Spülkanäle (13) mit jeweils einer zur Drehwelle (3) parallel orientierten Spülkanallängsachse (16) gleichverteilt um die Drehwelle (3) angeordnet sind, durch die bei Betrieb der Blutpumpe sich jeweils eine mit einer zur längs des Hauptströmungskanals (6) durchströmenden
Hauptdurchströmungsrichtung (HR) retrograd orientierte Spülkanalströmung (SR) ausbildet, und
dass die drei Spülkanäle (13) jeweils einen orthogonal zur Drehwelle (3) orientierten Spülkanalquerschnitt (17) besitzen, der jeweils nierenförmig ausgebildet ist und die Drehwelle (3) sektoral umfasst.

2. Blutpumpe nach Anspruch 1,
**dadurch gekennzeichnet, dass** die nierenförmigen Spülkanalquerschnitte (17) jeweils von einem Umfangsrand umfasst sind, der eine radial äußere, von der Drehwelle abgewandte konvexe Umfangsrandkontur und eine radial innere, zur Drehwelle zugewandte konkave Umfangsrandkontur aufweist, und
dass die konkaven Umfangsrandkonturen der drei Spülkanalquerschnitte (17) auf einer zentrisch zur Drehwelle (3) angeordneten ersten virtuellen Kreislinie (18) liegen, und
dass die konvexen Umfangsrandkonturen der drei Spülkanalquerschnitte (17) auf einer zentrisch zur Drehwelle (3) angeordneten zweiten virtuellen Kreislinie (19) liegen.

3. Blutpumpe nach Anspruch 1 oder 2,
**dadurch gekennzeichnet, dass** die Anzahl n an Schaufelblättern 6 ist.

4. Blutpumpe nach Anspruch 2 oder 3,
**dadurch gekennzeichnet, dass** die Schaufelblätter (5) jeweils eine radiale Erstreckung besitzen, die sich in axialer Projektion auf die Schaufelanordnung (4) längs der Drehwelle (3) von der zweiten virtuellen Kreislinie (19) bis zu einer zentrisch zur Drehwelle (3) angeordneten dritten virtuellen Kreislinie (20) erstreckt, und
dass der Radius (R2) der zweiten virtuellen Kreislinie (19) kleiner als der Radius (R3) der dritten virtuellen Kreislinie (20) ist.

5. Blutpumpe nach Anspruch 2 oder 3,
**dadurch gekennzeichnet, dass** eine erste Gruppe (G1) von n/2 der Schaufelblätter (5) jeweils eine radiale Erstreckung besitzen, die sich in axialer Projektion auf die Schaufelanordnung (4) längs der Drehwelle (3) von der zweiten virtuellen Kreislinie (19) bis zu einer zentrisch zur Drehwelle (3) angeordneten dritten virtuellen Kreislinie (20) erstreckt, wobei der Radius (R3) der dritten Kreislinie (19) größer als der Radius (R2) der zweiten Kreislinie (19) ist,
dass eine zweite Gruppe (G2) von n/2 der Schaufelblätter (5) jeweils eine radiale Erstreckung besitzen, die sich in axialer Projektion auf die Schaufelanordnung (4) längs der Drehwelle (3) von der ersten virtuellen Kreislinie (19) bis zu der zentrisch zur Drehwelle (3) angeordneten dritten virtuellen Kreislinie (20) erstreckt, und
dass die Schaufelblätter der ersten und zweiten Gruppe (G1, G2) jeweils mit abwechselnder Reihenfolge um die Drehwelle (3) angeordnet sind.

6. Blutpumpe nach einem der Ansprüche 1 bis 5,
**dadurch gekennzeichnet, dass** das am Innengehäuse (8) angebrachte untere Lager (14) ein fest mit dem Innengehäuse (8) gefügtes, topfförmiges Inlayelement (21) vorsieht, in das das untere Ende (3u) der Drehwelle (3) drehbar und axialfest einmündet und das aus einem anderen Material gefertigt ist als das Innengehäuse (8).

7. Blutpumpe nach Anspruch 6,
**dadurch gekennzeichnet, dass** das Material des Inlayelementes (21) aus einer Keramik oder aus einem UHM-Kunststoff besteht.

8. Blutpumpe nach Anspruch 7,
**dadurch gekennzeichnet, dass** die Lagerhülse (15) aus dem gleichen Material besteht wie das Inlayelement (21).

9. Blutpumpe nach einem der Ansprüche 1 bis 8,
**dadurch gekennzeichnet, dass** die Schaufelanordnung (4) eine Deckscheibe (22) vorsieht, die den Hautpdurchströmungskanal (6) mit dem Pumpengehäuse (1) begrenzt.

10. Blutpumpe nach Anspruch 9,
**dadurch gekennzeichnet, dass** die Deckscheibe (22) eine geradkegelstumpfförmige Oberfläche aufweist, die dem Pumpengehäuse zugewandt orientiert ist.

## Claims

1. A blood pump with a pump housing (1) comprising a bottleneck-shaped flow inlet region (2), in which there is arranged a blade assembly (4) that is mounted for conjoint rotation on a rotatably mounted rotary shaft (3), encloses a main flow channel (6) together with the pump housing (1), through which a blood flow passes in the main flow direction (HR) when the blood pump is in operation, and has a number n of blades (5), which are oriented radially to the rotary shaft, each have a flat shape, and are arranged about the rotary shaft (3) equidistantly to one another, and a hollow-cylindrical pump housing portion (7) which adjoins the bottleneck-shaped flow inlet region (2) downstream thereof along a flow outlet (1") oriented essentially orthogonally to the flow inlet, and in which a rotary motor (9) is arranged within an inner housing (8), said rotary motor (9) being operatively connected to the blade assembly (4) in order to rotate same via a magnetic coupling (10), and the inner housing (8) together with the pump housing (1) encloses a flow channel (11), which continues the main flow channel (6), and together with the blade assembly (4) encloses a secondary channel (12), which fluidically communicates with the main flow channel (6), said secondary channel being fluidically connected to at least one flushing channel (13) which passes through the blade assembly (4) and opens into the bottleneck-shaped flow inlet region (2), and the inner housing (8) additionally provides a lower bearing (14) facing the blade assembly (4) for receiving a lower end (3u) of the rotary shaft (3) in a rotatable manner, the upper end (3o) of the rotary shaft being mounted in a bearing sleeve (15) which is arranged in the bottleneck-shaped flow inlet region and which is indirectly secured to the pump housing (1), **characterised in that** three flushing channels (13), each of which has a flushing channel longitudinal axis (16) oriented parallel to the rotary shaft (3), are distributed evenly around the rotary shaft (3), by which, during operation of the blood pump, a flushing channel flow (SR) oriented retrograde to a main flow direction (HR) flowing along the main flow channel (6) is formed in each case, and
**in that** each of the three flushing channels (13) has a flushing channel cross-section (17) oriented orthogonally to the rotary shaft (3), said cross-sections (17) each being kidney-shaped and surrounding the rotary shaft (3) in sectors.

2. The blood pump according to claim 1,
**characterised in that** the kidney-shaped flushing channel cross-sections (17) are each enclosed by a peripheral edge which has a radially outer convex peripheral edge contour facing away from the rotary shaft and a radially inner concave peripheral edge contour facing towards the rotary shaft, and
**in that** the concave peripheral edge contours of the three flushing channel cross-sections (17) lie on a first virtual circular line (18) arranged centrically to the rotary shaft (3), and
**in that** the convex peripheral edge contours of the three flushing channel cross-sections (17) lie on a second virtual circular line (19) arranged centrically to the rotary shaft (3).

3. The blood pump according to claim 1 or 2,
**characterised in that** the number n of blades is 6.

4. The blood pump according to claim 2 or 3,
**characterised in that** the blades (5) each have a radial extent which, in axial projection onto the blade assembly (4), extends along the rotary shaft (3) from the second virtual circular line (19) to a third virtual circular line (20) arranged centrically to the rotary shaft (3), and
**in that** the radius (R2) of the second virtual circular line (19) is smaller than the radius (R3) of the third virtual circular line (20).

5. The blood pump according to claim 2 or 3,
**characterised in that** a first group (G1) of n/2 of the blades (5) each have a radial extent which, in axial projection onto the blade assembly (4), extends along the rotary shaft (3) from the second virtual circular line (19) to a third virtual circular line (20) arranged centrically to the rotary shaft (3), the radius (R3) of the third circular line (19) being greater than the radius (R2) of the second circular line (19),
**in that** a second group (G2) of n/2 of the blades (5) each have a radial extent which, in axial projection onto the blade assembly (4), extends along the rotary shaft (3) from the first virtual circular line (19) to the third virtual circular line (20) arranged centrically to the rotary shaft (3), and
**in that** the blades of the first and second groups (G1, G2) are each arranged in alternating order around the rotary shaft (3).

6. The blood pump according to one of claims 1 to 5,
**characterised in that** the lower bearing (14) attached to the inner housing (8) provides a pot-shaped inlay element (21) which is fixedly joined to the inner housing (8) and into which the lower end (3u) of the rotary shaft (3) opens in a rotatable and axially fixed manner and is made of a different material than that of the inner housing (8).

7. The blood pump according to claim 6,
**characterised in that** the material of the inlay element (21) consists of a ceramic or of a UHM plastic.

8. The blood pump according to claim 7,
**characterised in that** the bearing sleeve (15) is made of the same material as the inlay element (21).

9. The blood pump according to one of claims 1 to 8,
**characterised in that** the blade assembly (4) provides a cover disc (22) which delimits the main flow channel (6) with the pump housing (1).

10. The blood pump according to claim 9,
**characterised in that** the cover disc (22) has a straight frustoconical surface oriented facing towards the pump housing.

## Revendications

1. Pompe à sang, pourvue d'un corps de pompe (1), qui comprend une zone (2) d'entrée d'écoulement en forme de col de bouteille, sur laquelle est placé un ensemble d'aubes (4) monté de manière solidaire en rotation sur un arbre rotatif (3) logé de manière rotative, qui inclut avec le corps de pompe (1) une conduite (6) de circulation principale, que traverse un flux sanguin dans la direction (HR) de circulation principale lorsque la pompe à sang fonctionne et qui détient un nombre n de pales (5) d'aube, orienté de manière radiale par rapport à l'arbre rotatif, également conçues à surface plane, qui sont placées de manière équidistante les unes par rapport aux autres autour de l'arbre rotatif (3) et qui comporte un segment (7) de corps de pompe en forme de cylindre creux, se raccordant en aval sur la zone (2) d'entrée d'écoulement en forme de col de bouteille, le long duquel est placée une sortie d'écoulement (1"), orientée sensiblement de manière orthogonale par rapport à l'entrée d'écoulement et dans lequel, à l'intérieur d'un corps intérieur (8) est placé un moteur de rotation (9), qui par l'intermédiaire d'un accouplement magnétique (10) est en liaison active avec l'ensemble d'aubes (4), pour l'entraînement en rotation de celui-ci et le corps intérieur (8) inclut avec le corps de pompe (1) une conduite (11) de débit prolongeant la conduite (6) de circulation principale, ainsi qu'avec l'ensemble d'aubes (4) une conduite (12) secondaire qui est en communication fluidique avec la conduite (6) de circulation principale, qui est en liaison fluidique avec au moins une conduite (13) de rinçage, pénétrant à travers l'ensemble d'aubes (4) et débouchant dans la zone (2) d'entrée d'écoulement en forme de col de bouteille et en ce que le corps intérieur (8) prévoit par ailleurs en face de l'ensemble d'aubes (4) un palier (14) inférieur, destiné à la réception déplaçable en rotation d'une extrémité inférieure (3u) de l'arbre rotatif (3), dont l'extrémité supérieure (3o) est logée dans une douille de palier (15) placée dans la zone d'entrée d'écoulement en forme de col de bouteille, qui est indirectement fixé sur le corps de pompe (1),
**caractérisée en ce que** trois conduites (13) de rinçage, pourvues chacune d'un axe (16) de conduite de rinçage orienté à la parallèle de l'arbre rotatif (3), à travers lesquelles, lorsque la pompe à sang fonctionne se constitue chaque fois un écoulement (SR) de conduite principale, orienté de manière rétrograde avec une direction (HR) de circulation principale circulant le long de la conduite (6) de circulation principale sont placées en distribution régulière autour de l'arbre rotatif (3) et
**en ce que** les trois conduites (13) de rinçage détiennent chacune une section transversale (17) de conduite de rinçage, orientée chaque fois de manière orthogonale par rapport à l'arbre rotatif (3), qui est conçue chaque fois en forme de rein et qui englobe l'arbre rotatif (3) de manière sectorielle.

2. Pompe à sang selon la revendication 1,
**caractérisée en ce que** les sections transversales (17) de conduite de rinçage en forme de rein sont englobées chacune par un bord périphérique qui comporte un contour de bord périphérique convexe, extérieur en direction radiale, opposé à l'arbre rotatif et un contour de bord périphérique concave, intérieur en direction radiale, qui fait face à l'arbre rotatif et
**en ce que** les contours concaves de bord périphérique des trois sections transversales (17) de la conduite de rinçage se situent sur une première ligne circulaire (18) virtuelle, placée de manière centrée par rapport à l'arbre rotatif (3) et
**en ce que** les contours convexes de bord périphérique des trois sections transversales (17) de conduite de rinçage se situent sur une deuxième ligne circulaire (19) virtuelle, placée de manière centrée par rapport à l'arbre rotatif (3).

3. Pompe à sang selon la revendication 1 ou 2,
**caractérisée en ce que** le nombre n de pales d'aube est de 6.

4. Pompe à sang selon la revendication 2 ou 3,
**caractérisée en ce que** les pales (5) d'aube détiennent chacune une extension radiale, qui en projection axiale sur l'ensemble d'aubes (4) s'étend le long de l'arbre rotatif (3) de la deuxième ligne circulaire (19) virtuelle jusqu'à une troisième ligne circulaire (20) virtuelle, placée de manière centrée par rapport à l'arbre rotatif (3) et
**en ce que** le rayon (R2) de la deuxième ligne circulaire (19) virtuelle est inférieur au rayon (R3) de la troisième ligne circulaire (20) virtuelle.

5. Pompe à sang selon la revendication 2 ou 3,
**caractérisée en ce qu'**un premier groupe (G1) de n/2 des pales (5) d'aube détiennent chacune une extension radiale qui en projection axiale sur l'ensemble d'aubes (4) de l'arbre rotatif (3) s'étend de la deuxième ligne circulaire (19) virtuelle jusqu'à une troisième ligne circulaire (20) virtuelle, placée de manière centrée par rapport à l'arbre rotatif (3), le rayon (R3) de la troisième ligne circulaire (19) étant supérieur au rayon (R2) de la deuxième ligne circulaire (19),
**en ce qu'**un deuxième groupe (G2) de n/2 des pales (5) d'aube détiennent chacune une extension radiale qui en projection axiale sur l'ensemble d'aubes (4) de l'arbre rotatif (3) s'étend de la première ligne circulaire (19) virtuelle jusqu'à la troisième ligne circulaire (20) virtuelle, placée de manière centrée par rapport à l'arbre rotatif (3) et
**en ce que** les pales d'aube du premier et du deuxième groupes (G1, G2) sont placées chacune selon une séquence alternée autour de l'arbre rotatif (3).

6. Pompe à sang selon l'une quelconque des revendications 1 à 5,
**caractérisée en ce que** le palier (14) inférieur monté sur le corps intérieur (8) prévoit un élément incrusté (21) en forme de pot, fixement assemblé avec le corps intérieur (8), dans lequel l'extrémité inférieure (3u) de l'arbre rotatif (3) s'embouche da manière rotative et fixe en direction axiale et qui est fabriqué dans un autre matière que le corps intérieur (8).

7. Pompe à sang selon la revendication 6,
**caractérisée en ce que** la matière de l'élément incrusté (21) consiste dans une céramique ou dans une matière plastique UHM.

8. Pompe à sang selon la revendication 7,
**caractérisée en ce que** la douille de palier (15) consiste dans la même matière que l'élément incrusté (21).

9. Pompe à sang selon l'une quelconque des revendications 1 à 8,
**caractérisée en ce que** l'ensemble d'aubes (4) prévoit un disque de recouvrement (22) qui délimite la conduite (6) de circulation principale avec le corps de pompe (1).

10. Pompe à sang selon la revendication 9,
**caractérisée en ce que** le disque de recouvrement (22) comporte une surface de forme tronconique droite, qui est orientée en faisant face au corps de pompe.
